(19) Europäisches Patentamt

European Patent Office

Office européen des brevets

(11) **EP 0 834 344 A2**

(12) **EUROPÄISCHE PATENTANMELDUNG**

(43) Veröffentlichungstag:
08.04.1998 Patentblatt 1998/15

(51) Int. Cl.⁶: **B01D 53/04**, B01D 53/56

(21) Anmeldenummer: 97109046.9

(22) Anmeldetag: 04.06.1997

(84) Benannte Vertragsstaaten:
**AT BE DE ES FR GB IT NL**

(30) Priorität: **14.06.1996 DE 19623791**

(71) Anmelder:
**Linde Aktiengesellschaft
65189 Wiesbaden (DE)**

(72) Erfinder:
• **Langebach, Hans-Peter, Dipl.-Ing.
82031 Grünwald (DE)**

• **Baur, Karl, Dr.-Ing.
82065 Baierbrunn (DE)**
• **Wenning, Ulrike, Dr.-Ing.
85757 Karlsfeld (DE)**
• **Neumann, Hartmut
80797 München (DE)**

(74) Vertreter: **Kasseckert, Rainer
Linde Aktiengesellschaft,
Zentrale Patentabteilung
82049 Höllriegelskreuth (DE)**

(54) **Verfahren zur Entfernung von Stickoxiden aus Gasen**

(57)     Die Erfindung betrifft ein Verfahren zur selektiven Entfernung von Stickoxiden aus einem kohlenwasserstoffhaltigen Trägergas. Erfindungsgemäß werden die Stickoxide durch Chemisorption an Metalloxiden entfernt, ohne daß unerwünschte Nebenreaktionen auftreten.

Das Verfahren enthält ein oder mehrere Reaktorbetten, die bevorzugt bei 10 bis 40°C betrieben und mit Stickstoff einer Temperatur von 130 bis 170°C regeneriert werden.

Das Verfahren kann vorteilhaft zur Entfernung von Stickoxiden aus einem olefinreichen Gas, beispielsweise aus dem Abgas einer FCC-Anlage, angewendet werden, insbesondere wenn das Gas anschließend einer Olefingewinnung zugeführt wird.

Das Trägergas kann insbesondere ungesättigte Kohlenwasserstoffe und darüber hinaus Wasserstoff und Kohlenmonoxid enthalten, auch Sauerstoff, dessen Konzentration dann bevorzugt zwischen 100 und 5000 mol ppm liegt. Die Metalloxide sind bevorzugt aus Metallen der 6. bis 8. Nebengruppe gebildet, wobei Braunstein ($MnO_2$) als Metalloxid besonders bevorzugt wird.

EP 0 834 344 A2

**Beschreibung**

Die Erfindung betrifft ein Verfahren zur selektiven Entfernung von Stickoxiden aus einem kohlenwasserstoffhaltigen Trägergas.

Die Notwendigkeit, Stickoxide aus einem solchen Gasstrom zu entfernen, besteht beispielsweise bei einer Ethylenanlage, wenn diese Anlage auch Olefine aus einer FCC (Fluidized Catalytic Cracking) Anlage verarbeiten soll. In der FCC-Anlage werden langkettige Kohlenwasserstoffe aufgebrochen, wobei leichte Olefine entstehen. Bei der Katalysatorregenerierung der FCC-Anlage mittels Luft entstehen Stickoxide, wie NO und $NO_2$. Zusätzlich ist freier Sauerstoff vorhanden. Insbesondere bei tiefen Temperaturen unter 0°C, wie sie beispielsweise im Tieftemperaturteil einer Ethylenanlage auftreten, findet mit dem zumindest in Spuren immer vorhandenen Sauerstoff eine Umsetzung des NO zu $N_2O_3$ mit einem Siedepunkt bei -102°C und zu $N_2O_4$ mit einem Siedepunkt bei -11°C statt, und diese Stickoxide können mit olefinischen Wasserstoffen mit konjugierten Doppelbindungen, beispielsweise mit Butadien, unter Bildung von explosiven Harzen reagieren und sich in der Tieftemperaturserlegung der Olefinanlage anreichern. Diese Problematik ist ausführlich in der Druckschrift: H. Bauer, Nicht kryogene und gefahrlose Entmethanisierung von FCC-Restgasen, Linde-Berichte aus Technik und Wissenschaft 72 (1994) Seite 15-18 beschrieben.

Andererseits kann die Bildung von NO in einer FCC-Anlage nicht mit wirtschaftlich sinnvollem Aufwand vermieden werden. Bisher bekannt sind Verfahren zur Gewinnung von olefinischen Kohlenwasserstoffen durch Absorption in Waschmitteln unter Vermeidung tiefer Temperaturen oder dem Fachmann bekannte kryogene Verfahren, die zur Vermeidung der Anreicherung explosiver Harze aufwendige analytische und betriebstechnische Maßnahmen erfordern, wobei zusätzlich notwendige Investitionen das Verfahren verteuern. Ein Verfahren zur selektiven Abtrennung von Stickoxiden aus FCC-Restgas war bisher nicht bekannt.

Aufgabe der Erfindung ist es deshalb, selektiv Stickoxide aus kohlenwasserstoffhaltigem Gas, beispielsweise aus FCC-Abgas, zu entfernen, um bewährte kryogene Verfahren zur Olefingewinnung einsetzen zu können.

Diese Aufgabe wird erfindungsgemäß gelöst von einem Verfahren, bei dem die Stickoxide durch Chemisorption an Metalloxiden entfernt werden. Besonders vorteilhafte Ausführungen der Erfindung sind Gegenstand von Unteransprüchen.

Die Entfernung von Stickoxiden aus Gasen ist an sich bekannt. In der Druckschrift: Hamid Arastoopour und Hossein Hariri, $NO_x$ Removal with High - Capacity Metall Oxides in the Pressence of Oxygen, Ind. Eng. Chem. Process Des. Dev. 1981, 20, Seite 223-228 ist ein Verfahren nach dem Oberbegriff des Anspruchs 1 beschrieben. Es soll zur Reinigung von Rauchgasen verwendet werden. Deshalb werden Gase mit hohem Sauerstoffgehalt von 3 % und hoher Stickstoffmonoxidkonzentration von 680 mol ppm bei Anwesenheit von Wasserdampf mit Braunstein als Sorbens untersucht und unter diesen Bedingungen eine nur geringe Beladbarkeit gefunden. Regeneriert wird thermisch bei 500°C. Die Chemisorption von Stickstoffmonoxid an Braunstein wird auf folgende chemische Reaktionsgleichung zurückgeführt:

$$MnO_2 + 2NO + O_2 \leftrightarrow Mn\,(NO_3)_2$$

mit einem Gleichgewicht, das bei hohen Temperaturen auf der linken Seite der Gleichung liegt, wodurch thermisches Regenerieren ermöglicht wird.

Neben der bei Arastoopour genannten Reaktion ist auch die folgende chemische Reaktion des Braunsteins mit Stickstoffdioxid möglich:

$$MnO_2 + 2NO_2 \leftrightarrow Mn\,(NO_3)_2$$

Den Einsatz eines kohlenwasserstoffhaltigen, insbesondere Olefine und auch Wasserstoff enthaltenden Gases in einem Reaktor mit $MnO_2$ hat der Fachmann bisher nicht erwogen, da die katalytische Wirkung von $MnO_2$ die Hydrierung ungesättigter Kohlenwasserstoffe mit dem immer vorhandenen Wasserstoff erwarten ließ. Die Wärmetönung von immer in Spuren vorhandenen Komponenten wie Kohlenmonoxid und anderen kann bei der Reaktion mit Sauerstoff zu weiteren unkontrollierbaren und unerwünschten Reaktionen führen. Laborversuche mit dem Katalysator T 2525 der Fa. Südchemie, der aus $MnO_2$ auf Aluminiumoxid besteht, und mit anderen $MnO_2$-haltigen Katalysatoren mit einem anderen Trägermaterial, führen nun zu dem überraschenden Ergebnis, daß auch Spuren von NO mit hoher Ausbeute ohne die vermuteten unerwünschten chemischen Nebenreaktionen absorbiert werden.

Bevorzugt enthält das Trägergas auch Spuren von Sauerstoff, da es die Chemisorption von Stickoxiden begünstigt. Überraschenderweise haben 0,3 mol % Sauerstoff zu keinen Nebenreaktionen geführt. Dies ist überraschend, da dem Fachmann $MnO_2$ als Katalysator zur Kohlenwasserstoff-Oxidation, auch schon bei Temperaturen unter 100°C, bekannt ist.

Eine besonders bevorzugte Anwendung stellt die Entfernung von Stickoxiden aus einem olefinreichen Raffinerieabgas dar. Vorteilhaft ist beispielsweise eine Anwendung bei der Entstickung von FCC-Gasen einer Raffinerie, da diese Gase über einen hohen Anteil an Kohlenwasserstoff-Wertprodukten verfügen.

Im erfindungsgemäßen Verfahren können vorteilhaft Metalloxide aus Metallen der 6. bis 8. Nebengruppe des Periodensystems, insbesondere handelsübliche Katalysatoren, verwendet werden. Sie enthalten vorzugsweise Braunstein oder ein mit Braunstein beschichtetes Trägermaterial mit großer spezifischer Oberfläche, also beispielsweise in körniger Form als Schüttung. Im vom Verfahrensgas durchströmten Reaktorbett findet die Chemisorption der Stickoxide an $MnO_2$ statt, das hierbei zu $Mn(NO_3)_2$ umgesetzt wird. Deshalb muß das Reaktorbett vor dem Durchbruch von Stickoxiden regeneriert werden. Vorteilhafterweise wird zu diesem Zweck das Reaktorbett mit warmen in diesem Zusammenhang nicht reaktiv wirkendem Gas, vorzugsweise Stickstoff, mit einer Temperatur von 100 bis 300°C, vorzugsweise 130 bis 170°C, durchströmt.

Für die Absorption ist eine Betriebstemperatur zwischen 5 und 100°C vorteilhaft, besonders vorteilhaft ist eine solche zwischen 10 und 40°C.

Die Ausgestaltung der Vorrichtung zur Entfernung von Stickoxiden aus Gasen hängt von Randbedingungen der Anwendung ab. Zur Entfernung von Spuren von Stickoxiden in Größenordnungen unter 0,1 mol ppm, oder wenn Stickoxide sich nur sporadisch im Trägergas befinden oder wenn entsprechend lange Pausen im Betrieb dies erlauben, kann mit Vorteil ein einziges Reaktorbett eingesetzt werden.

Wenn ein kontinuierlicher Betrieb der Vorrichtung notwendig ist, werden vorteilhaft mehrere, mindestens jedoch zwei, Reaktorbetten eingesetzt. Mindestens ein Reaktorbett übernimmt hierzu die Chemisorption, das oder die anderen Reaktorbetten werden regeneriert. Die eingesetzten Reaktorbetten übernehmen nacheinander die Chemisorption, so daß eine kontinuierliche Stickoxidentfernung ermöglicht wird.

Beispiel 1

Mit einem Katalysator der Fa. Südchemie, Typ 2525 mit $MnO_2$ auf $Al_2O_3$, ergeben Laborversuche bei Raumtemperatur und Atmosphärendruck und mit den Daten:

| | |
|---|---|
| Raumgeschwindigkeit | $4000\ h^{-1}$ |
| NO-Gehalt | 20 mol ppb |
| $O_2$-Gehalt | 520 mol ppm |
| gesättigte $C_1$-$C_3$-Kohlenwasserstoffe | ca. 70 mol % |
| $H_2$ | 25 mol % |
| CO | 2 mol % |
| Rest | $N_2$ |

nach einer Laufzeit von mehr als 130 h eine NO-Entfernung von 85 %.

Beispiel 2

Analoge Versuche mit Ethylen und Propylen anstelle gesättigter Kohlenwasserstoffe führen zur gleichen NO-Entfernung wie im Beispiel 1. Es werden keine Nebenreaktionen durch Oxidation von Kohlenmonoxid und Kohlenwasserstoffen oder eine Hydrierung von Olefinen festgestellt.

Alle Versuche zur Absorption von Stickoxiden wurden bei Atmosphärendruck und bei einer Raumgeschwindigkeit von etwa $4000\ h^{-1}$ vorgenommen. Die Anwendung von höheren Drücken läßt eine Verbesserung der Stickoxidentfernung erwarten.

**Patentansprüche**

1. Verfahren zur selektiven Entfernung von Stickoxiden aus einem kohlenwasserstoffhaltigen Trägergas, **dadurch gekennzeichnet**, daß die Stickoxide durch Chemisorption an Metalloxiden entfernt werden, ohne daß unerwünschte Nebenreaktionen auftreten.

2. Verfahren nach Anspruch 1, dadurch gekennzeichnet, daß das Trägergas an Kohlenwasserstoffen insbesondere auch ungesättigte Kohlenwasserstoffe und darüber hinaus Wasserstoff und Kohlenmonoxid enthält.

3. Verfahren nach Anspruch 1 oder 2, dadurch gekennzeichnet, daß das Trägergas auch Sauerstoff enthält, vorzugsweise zwischen 100 und 5000 mol ppm.

4. Verfahren nach einem der Ansprüche 1 bis 3, dadurch gekennzeichnet, daß die Metalle, aus denen die Metalloxide gebildet sind, Metalle der 6. bis 8. Nebengruppe sind, vorzugsweise jedoch Mangandioxid ist, das besonders bevorzugt auf einem Trägermaterial wie Aluminiumoxid oder Siliziumoxid aufgebracht wird.

5. Verfahren nach einem der Ansprüche 1 bis 4, dadurch gekennzeichnet, daß die Stickoxidentfernung vorzugsweise bei einer Temperatur zwischen 0 und 100°C, besonders bevorzugt zwischen 10 und 40°C, und bevorzugt bei einem Druck zwischen 1 und 50 bar vorgenommen wird.

6. Verfahren nach einem der Ansprüche 1 bis 5, dadurch gekennzeichnet, daß die Stickoxidentfernung bevorzugt bei einer Raumgeschwindigkeit zwischen 300 und 12000 $h^{-1}$, besonders bevorzugt zwischen 500 und 8000 $h^{-1}$, vorgenommen wird.

7. Verfahren nach einem der Ansprüche 1 bis 6, das in einem oder mehreren Reaktorbetten, die Braunstein oder ein mit Braunstein beschichtetes Trägermaterial mit großer spezifischer Oberfläche, beispielsweise in körniger Form als Schüttung, enthalten, durchgeführt wird, wobei das Reaktorbett die Chemisorption der Stickoxide bewirkt und nach Erreichen einer Grenzbeladung regeneriert wird, dadurch gekennzeichnet, daß das zu regenerierende Reaktorbett von einem dabei nicht reaktiven Gas, vorzugsweise Stickstoff, durchströmt wird, dessen Temperatur bei 100 bis 300°C, vorzugsweise jedoch bei 130 bis 170°C liegt.

8. Verfahren nach Anspruch 7, dadurch gekennzeichnet, daß nur ein Reaktorbett eingesetzt wird, wenn Stickoxide sich in Spuren von weniger als 0,1 mol ppm oder nur kurzfristig im Trägergas befinden, oder wenn bei der Anwendung des Verfahrens Pausen im Betrieb das Regenerieren des Reaktorbettes ermöglichen.

9. Verfahren nach Anspruch 7, dadurch gekennzeichnet, daß mehrere, mindestens jedoch zwei, Reaktorbetten so geschaltet werden, daß ein kontinuierlicher Betrieb der Entfernung von Stickoxiden erreicht wird, indem die Reaktorbetten abwechselnd die Chemisorption der Stickoxide übernehmen, während gleichzeitig das oder die anderen Reaktorbetten regeneriert werden.

10. Anwendung des Verfahrens nach einem der Ansprüche 1 bis 9 bei der selektiven Entfernung von Stickoxiden aus olefinreichen Gasen aus thermischen und katalytischen Prozessen.

11. Anwendung nach Anspruch 10, dadurch gekennzeichnet, daß besagtes Gas aus einer FCC (Fluidized Catalytic Cracking) - Anlage einer Raffinerie stammt.

12. Anwendung nach Anspruch 10, dadurch gekennzeichnet, daß das olefinreiche Gas nach der selektiven Entfernung von Stickoxiden einer Olefingewinnung zugeführt wird.